# EUROPEAN PATENT SPECIFICATION

(11) **EP 3 573 597 B1**
(45) Date of publication and mention of the grant of the patent: **20.08.2025**
(21) Application number: 18703628.0
(22) Date of filing: 17.01.2018
(51) Int. Cl.: A61K 9/00, A61K 47/32, A61K 47/36

(54) **TEAR SUBSTITUTE**
TRÄNENFLÜSSIGKEITSSUBSTITUT
LARMES ARTIFICIELLES

(30) Priority: 30.01.2017 IT 201700009786
(43) Date of publication of application: 04.12.2019
(73) Proprietor: Medivis S.r.l., 95030 Tremestieri Etneo, Catania (IT)
(72) Inventor: MANGIAFICO, Sebastiano, 95030 Tremestieri Etneo, Catania (IT)
(74) Representative: Long, Giorgio
(86) International application number: PCT/IB2018/050285
(87) International publication number: WO 2018/138602

(56) References cited:
- US-A- 5 876 744
- US-A1- 2004 137 079
- US-A1- 2008 050 335

## Description

The present invention relates to a composition comprising PVA in amounts between 0.1 and 0.3% by weight, preferably 0.25%, characterized by an optimal wettability for use as a tear substitute.

### Background art

The cornea wettability is a feature to be kept in strict consideration when developing a tear substitute. The cornea surface is covered by a hydrophilic extracellular matrix consisting of glycoproteins. In the treatment of patients with dry eye syndrome, those eye drops should be preferred that better wet not only the corneal surface with an intact mucin layer, but also those areas where the mucin layer is damaged or absent and therefore appear to be more hydrophobic.

The tear substitutes available today do not have these desired wettability features. Formulations with optimal wettability exist, achieved however with the use of surfactants, preferably with low molecular weight. Such formulations are disadvantageous, given the heavy toxic effects exerted by surfactants on the corneal epithelium.

US4883658 describes a formulation comprising partially hydrolyzed PVA and fully hydrolyzed PVA, wherein said formulation is able to lead to a significant lowering of the surface tension of the aqueous solution and to a significant increase in the wettability of the hydrophobic surfaces.

WO2008011836 describes the synergistic effect on the viscosity obtained by combining hyaluronic acid and PVA, wherein the used percentages of PVA are between 0.25% and 4%.

The wettability of a solid (cornea) by a liquid (eye drops) can be described and quantified by referring to phenomena such as, for example, the capillarity and the contact angle. To study the properties of thin layers of liquids on the surfaces of solids, the study of the contact angle is considered the most useful approach. The contact angle of a liquid drop on a smooth surface is determined by the solid-gas, solid-liquid and liquid-gas interfacial tension. The contact angle has a value equal to zero (θ = 0° complete wettability) when the sum between the liquid surface tension and the solid-liquid interfacial tension is equal to or less than the solid surface tension. In general, the greater the contact angle, the lower the liquid tendency to wet the solid. Very often the liquid shows no special affinity for the solid and only weak forces such as the Van der Waals and London forces operate at the solid-liquid interface. If the liquid has the same surface tension as the solid, it will spread spontaneously, completely wetting the solid surface (θ = 0°). Also in the case of a negligible liquid-solid interfacial tension, an increase in the liquid surface tension is linearly proportional to the cosine of the contact angle (Zisman W. A. Relation of the Equilibrium Contact Angle to Liquid and Solid Constitution. In F.M. Fowkes (Ed.) Contact Angle, Wettability and Adhesion. (Advances in Chemistry, Series 43). Washington, D.C.: American Chemical Society, 1964 page 15).

Once the hydrophobic reference surfaces have been defined, a comparison of the contact angles obtained with various different formulations is therefore an indication of the cornea wettability degree. It should be considered that the absolute value of the contact angles on inert surfaces must be considered higher than the one obtainable for the same liquid (formulation) on biological surfaces, wherein the liquid-solid interfacial tensions are affected by the presence of hydrogen bonds.

It is an object of the present invention to provide a tear substitute with great ability to wet the corneal surface.

### Description of the invention

### Description of the drawings

Figure 1: flow curve viscosity *vs* shear rate of exemplary formulations according to the present invention.
Figure 2: elastic modulus and viscous modulus curve vs frequency of exemplary formulations according to the present invention.
Figure 3: curves of the contact angle over the time measured on plexiglass of exemplary formulations according to the present invention.
Figure 4: photograph showing the contact angle on a Kapton surface of the formulation HA1 according to the present invention (panel A) and of the comparative composition V1 (panel B).

Several eye drops were compared using two reference surfaces: plexiglass (polymethyl methacrylate) and kapton (polyimide). Both surfaces have the same surface tension but the kapton surface has a greater number of heteroatoms and therefore has a greater ability to establish hydrogen bonds.

The cornea superficial tension in the presence of mucus is about 40 mN/m (Sharma A, Biophis. Chem. 1993, 47: 87-99). This result is in accordance with the surface tension obtained according to the Zisman method in previous studies on the cornea wettability and surface tension (Holly FJ and Lemp MA, Exp. Eye Res. 1971, 11:239). The surface tension of plexiglass and kapton is about 38-40 mN/m (Jarvis NL et al., Surface activity at organic liquid-air interfaces: the effect of partially fluorinated additives on the wettability of solid polymers. In F.M. Fowkes Contact Angle, Wettability and Adhesion. Advances in Chemistry, Series 43. Washington, D.C.: American Chemical Society, 1964 page 317). The surfaces have therefore been selected because they well mimic the corneal surface.

Polyvinyl alcohol (PVA) is a polymer which, when placed in water, lowers the surface tension thereof and is therefore used to increase wettability on hydrophobic surfaces. PVA 4-88 and PVA 28-99 are widely used in ophthalmology. The first number of the pair of numbers used to identify the type of PVA refers to the viscosity of a 4% solution and roughly indicates the polymerization degree. The second number refers to the percentage of free hydroxyl groups (hydrolysates), being the remaining acetylated groups.

The present invention relates to tear substitutes comprising: hyaluronic acid, mineral salts, potassium citrate, glycerol, and PVA in an amount ranging from 0.1 to 0.3% by weight, preferably 0.25% w/w.

In a preferred embodiment, said PVA is selected from the group comprising PVA 4-88, PVA 28-99.

In a further embodiment, said tear substitute comprises: PVA 0.25 g/100 g, sodium hyaluronate 0.050-0.200 g/100 g, NaCl 0.150-0.400 g/100 g, Na₂HPO₄*12H₂O 0.200-0.600 g/100 g, potassium citrate*H₂O 0.010-0.060 g/100 g, KCl 0.050-0.200 g/100 g, CaCl₂*2H₂O 0.005-0.02 g/100 g, MgCl₂*6H₂O 0.005-0.02 g/100 g, Glycerol 0.050-0.900 g/100 g.

In a particularly advantageous form, said tear substitute comprises, in 100 g: PVA 0.25 g, sodium hyaluronate 0.180 g, NaCl 0.279 g, Na₂HPO₄*12H₂O 0.322 g, potassium citrate*H₂O 0.031 g, KCl 0.103 g, CaCl₂*2H₂O 0.0089 g, MgCl₂*6H₂O 0.0092 g, Glycerol 0.200 g, H₂O q.s. The formulations according to the present invention have been tested and compared with compositions glycerol and PVA (V1)-free or with glycerol and PVA-free (V1G).

Table 1 shows the study formulations.

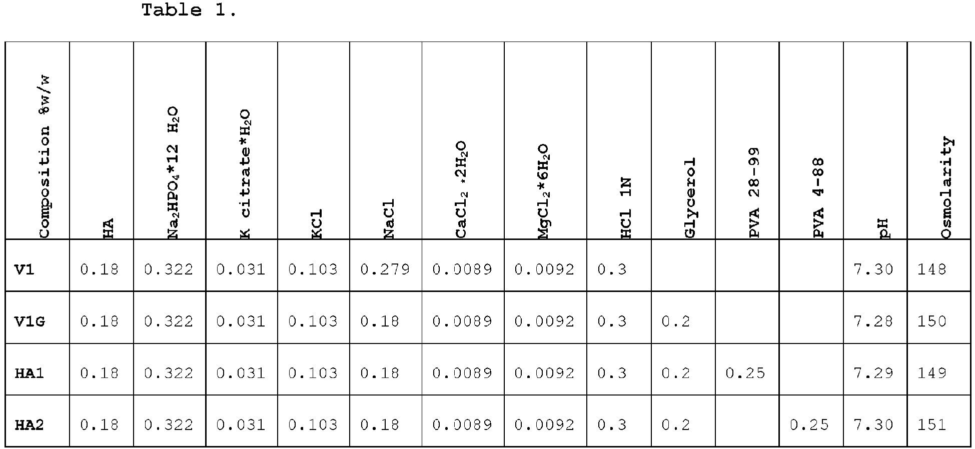

After verifying that all the formulations showed roughly the same values of osmolarity and pH, the most complex evaluations of the rheological characteristics have been analyzed, in particular the flow curves, the elastic moduli G' and the viscous modulus G" were evaluated. The elastic moduli G' and G" are shown only for V1 and HA2 being these representatives also for V1G and HA1.

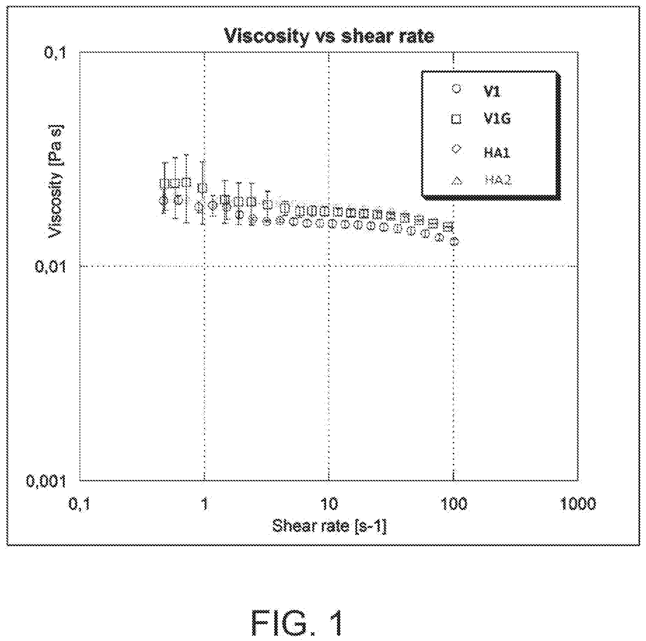

Figure 1 shows the viscosity values for the formulations HA1 and HA2 according to the present invention. For comparative purposes, the viscosity values were measured for the comparative formulations V1 and V1G. The viscosities of all the preparations are between 10 and 30 mPa·s.

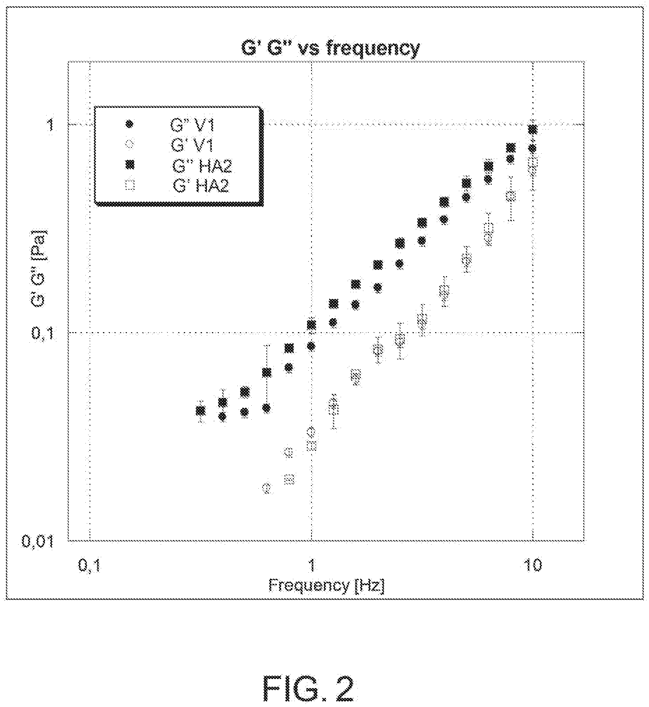

Figure 2 shows the values of elastic modulus G' and viscous modulus G'**.** In the frequency interval of 0.5 and 10 Hz in the experiment of Figure 2 it is observed that the elastic (G') and viscous (G") moduli of the two formulations, the control V1 and the formulation according to the present invention HA2, do not show significant differences indicating that the addition of PVA in the formulation does not interfere with the rheological behavior of the hyaluronic acid which is maintained.

Finally, the contact angle was evaluated, followed by the wettability on a plexiglass surface and on a kapton surface, both with a surface tension of about 40 mN/m and therefore intended as models of the corneal surface. The angle was measured with the Contact Angle System OCAE15 instrument using the sessile drop method, with a dispensed volume of 3 µl.

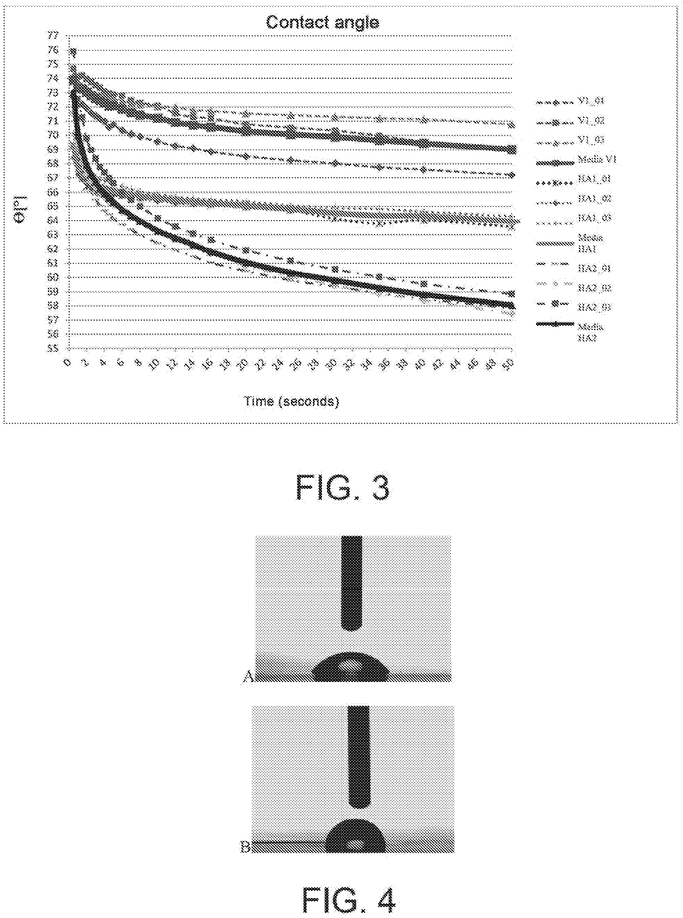

The results are shown in Figure 3. The formulations HA1 and HA2 according to the present invention immediately showed a better wettability of the plexiglass surface with respect to the comparative formulation V1. From the results obtained on plexiglass results a fair difference in the variation of the contact angle over the time. Taking the equilibrium contact angle at 50 sec., it results: θ = 69 for V1, θ = 58 for HA2 and θ = 64 for HA1. The formulations HA2 and HA1 according to the present invention, all else being equal, have the advantage of better wetting hydrophobic surfaces with superficial tensions similar to the cornea, showing how minimum doses of PVA such as those used herein are surprisingly able of improving the wettability features, without influencing other composition features of the composition containing them.

Therefore, the formulations according to the present invention are surprisingly advantageous.

### Chemical stability of the formulations

The formulations HA2 and HA1 were packaged in sterile vials and stored at temperatures between 25 °C and 40 °C for a stability study. In particular, the HA2 formulation containing PVA with a hydrolysis degree of 88% was formulated with greater amounts of citrate buffer to avoid pH variations, in fact PVA 4-88, due to the acetyls hydrolysis, decreases the pH of the ophthalmic composition. Table 2 and Table 3 show the results of the stability study related to the pH variation of the formulations V1, HA1 and HA2.

**Table 2.**

| Stability 25°C | | T₀ | T = 15days | T = 1 month | T = 2 months | T = 3 months | T = 4 months |
|---|---|---|---|---|---|---|---|
| **HA2** | pH | 7.29 | 7.28 | 7.26 | 7.20 | 7.17 | 7.12 |
| **+ citrate 0.06%** | pH | 7.24 | 7.21 | 7.22 | 7.18 | 7.15 | 7.10 |
| **+ citrate 0,1%** | pH | 7.27 | 7.25 | 7.24 | 7.20 | 7.17 | 7.12 |
| **+ citrate 0.15%** | pH | 7.24 | 7.22 | 7.19 | 7.18 | 7.14 | 7.10 |
| **V1** | pH | 7.30 | 7.28 | 7.29 | 7.29 | 7.28 | 7.29 |
| **HA1** | pH | 7.32 | 7.30 | 7.31 | 7.30 | 7.29 | 7.30 |

**Table 3.**

| **Stability 40°C** | | **T zero** | **T= 15days** | **T=1 month** | **T=2 months** | **T=3 months** | **T=4 months** |
|---|---|---|---|---|---|---|---|
| **HA2** | pH | 7.29 | 7.14 | 6.99 | 6.85 | 6.70 | 6.63 |
| **+ citrate 0.06%** | pH | 7.24 | 7.12 | 7.01 | 6.90 | 6.79 | 6.66 |
| **+ citrate 0,1%** | pH | 7.27 | 7.14 | 7.04 | 6.91 | 6.83 | 6.70 |
| **+ citrate 0.15%** | pH | 7.24 | 7.13 | 7.03 | 6.90 | 6.90 | 6.77 |
| **V1** | pH | 7.30 | 7.29 | 7.28 | 7.28 | 7.27 | 7.28 |
| **HA1** | pH | 7.32 | 7.30 | 7.27 | 7.25 | 7.26 | 7.26 |

The obtained data show a relatively important pH variation for the compositions containing PVA 4-88. Vice versa, HA1 containing totally hydrolyzed PVA is stable at pH variation over the time.

### Contact angle of HA1 on Kapton surface

The contact angle measurements on Plexiglass showed an advantage in adding 0.25% PVA to the control composition. The measurements have great validity from a comparative point of view and without any doubt the difference is indicative of an advantage on cornea wettability. Considering that the values of the surface tensions experimentally determined with the Zisman method mainly consider the non-polar component of the surface tension and exclude the polar component which also has effect on wettability, a second hydrophobic surface, the Kapton, has been analyzed herein. This material has a greater number of heteroatoms and therefore has a greater chance to form hydrogen bonds. Figures 4 and Table 4 show respectively an indicative photograph and the average results obtained from 5 measurements of the contact angle after 2 seconds from the deposition of the drop on the Kapton surface. The instrument used was the Contact Angle System OCAE15 and the test type was the sessile drop method with a volume of 1 µl.

**Table 4.**

| | **V1** | **HA1** |
|---|---|---|
| | **Kapton** | **Kapton** |
| **Measurement 1** | **81.8** | 57.3 |
| **Measurement 2** | **78.4** | 58.8 |
| **Measurement 3** | **81.4** | 53.9 |
| **Measurement 4** | **83.3** | 57.7 |
| **Measurement 5** | **81.5** | 57.7 |
| **Average** | **81.3** | **57.1** |

The difference between the contact angle obtained with the formulation V1 and the formulation HA1 according to the present invention is even more evident than the one obtained with plexiglass. The contact angle of HA1 in the case of the Kapton has a lower value than the one expected for the Plexiglass due to the non-negligible contribution of the energy at the liquid-solid interface. Therefore, if the liquid-solid interface tension (polar interactions) can be ignored, as in plexiglass, a significant difference in contact angles is obtained. In the case of the Kapton, which has about the same surface tension as the Plexiglass, the differences in the contact angles are greater because the HA1 in which totally hydrolyzed PVA 28-99 is present, also originates polar interactions (as happening at the corneal level). The obtained results are comparable to what is observed on the cornea which, in addition to being hydrophobic, is rich in sites available to form hydrogen bonds.

Surprisingly, the solution described herein shows that the addition of glycerol and PVA at concentrations lower than those reported up to now in the literature (also patent literature) to known hyaluronic acid formulations has allowed to improve the wetting ability of a solid surface very similar to the corneal surface. Both 4-88 and 28-99 PVAs were formulated in a 0.25% concentration. Both PVAs were useful to improve the wettability on hydrophobic surfaces used as a model for the cornea. PVA 28-99 at a concentration of 0.25% provided a formulation with stable pH over time. Finally, the contact angle measurements on a hydrophobic surface able also of weak polar interactions, the Kapton, have further confirmed the ability of PVA 28-99 to significantly increase the wetting ability of hyaluronic acid formulations towards hydrophobic surfaces more similar to the cornea.

## Claims

1. A composition comprising: hyaluronic acid, mineral salts, citrate, glycerol, polyvinyl alcohol (PVA) between 0.1 and 0.3% by weight.

2. The composition according to claim 1, wherein said PVA is present in a concentration of 0.25% w/w.

3. The composition according to claim 1 or 2, wherein said citrate is potassium citrate and the PVA is selected from PVA 4-88 and PVA 28-99.

4. The composition according to one of claims from 1 to 3 comprising: PVA 0.25 g/100 g, sodium hyaluronate 0.050 - 0.200 g/100 g, NaCl 0.150-0.400 g/100 g, Na₂HPO₄*12H₂O 0.200-0.600 g/100 g, potassium citrate*H₂O 0.010-0.060 g/100 g, KCl 0.050-0.200 g/100 g, CaCl₂*2H₂O 0.005-0.02 g/100 g, MgCl₂*6H₂O 0.005-0.02 g/100 g, glycerol 0.050 - 0.900 g/100 g.

5. The composition according to one of claims from 1 to 4 comprising: PVA 0.25 g, sodium hyaluronate 0.180 g, NaCl 0.279 g, Na₂HPO₄*12H₂O 0.322 g, potassium citrate*H₂O 0.031 g, KCl 0.103 g, CaCl₂*2H₂O 0.0089 g, MgCl₂*6H₂O 0.0092 g, glycerol 0.200 g, H₂O qs.

6. The composition according to one of claims from 1 to 5 consisting in: PVA 0.25 g, sodium hyaluronate 0.180 g, NaCl 0.279 g, Na₂HPO₄*12H₂O 0.322 g, potassium citrate*H₂O 0.031 g, KCl 0.103 g, CaCl₂*2H₂O 0.0089 g, MgCl₂*6H₂O 0.0092 g, glycerol 0.200 g, H₂O qs.

7. The composition according to one of claims 1 to 6 for use as tear substitute.

## Patentansprüche

1. Zusammensetzung umfassend: Hyaluronsäure, Mineralsalze, Citrat, Glycerin, Polyvinylalkohol (PVA) in einer Menge zwischen 0,1 und 0,3 Gewichtsprozent.

2. Zusammensetzung nach Anspruch 1, wobei der PVA in einer Konzentration von 0,25 Gew.-% vorliegt.

3. Zusammensetzung nach Anspruch 1 oder 2, wobei das Citrat Kaliumcitrat ist und der PVA aus PVA 4-88 und PVA 28-99 ausgewählt ist.

4. Zusammensetzung nach einem der Ansprüche 1 bis 3, umfassend: PVA 0,25 g/100 g, Natriumhyaluronat 0,050 - 0,200 g/100 g, NaCl 0,150 - 0,400 g/100 g, Na₂HPO₄*12H₂O 0,200 - 0,600 g/100 g, Kaliumcitrat*H₂O 0,010 - 0,060 g/100 g, KCl 0,050 - 0,200 g/100 g, CaCl₂*2H₂O 0,005 - 0,02 g/100 g, MgCl₂*6H₂O 0,005 - 0,02 g/100 g, Glycerin 0,050 - 0,900 g/100 g.

5. Zusammensetzung nach einem der Ansprüche 1 bis 4, umfassend: PVA 0,25 g, Natriumhyaluronat 0,180 g, NaCl 0,279 g, Na₂HPO₄*12H₂O 0,322 g, Kaliumcitrat*H₂O 0,031 g, KCl 0,103 g, CaCl₂*2H₂O 0,0089 g, MgCl₂*6H₂O 0,0092 g, Glycerin 0,200 g, H₂O ad.

6. Zusammensetzung nach einem der Ansprüche 1 bis 5, bestehend aus: PVA 0,25 g, Natriumhyaluronat 0,180 g, NaCl 0,279 g, Na₂HPO₄*12H₂O 0,322 g, Kaliumcitrat*H₂O 0,031 g, KCl 0,103 g, CaCl₂*2H₂O 0,0089 g, MgCl₂*6H₂O 0,0092 g, Glycerin 0,200 g, H₂O ad.

7. Zusammensetzung nach einem der Ansprüche 1 bis 6 zur Verwendung als Tränenflüssigkeitssubstitut.

## Revendications

1. Une composition comprenant : acide hyaluronique, sels minéraux, citrate, glycérol, alcool polyvinylique (PVA) entre 0,1 et 0,3 % en poids.

2. La composition selon la revendication 1, dans laquelle ledit PVA est présent à une concentration de 0,25 % p/p

3. La composition selon la revendication 1 ou 2, dans laquelle ledit citrate est du citrate de potassium et le PVA est choisi parmi le PVA 4-88 et le PVA 28-99.

4. La composition selon l'une des revendications 1 à 3 comprenant : PVA 0,25 g/100 g, hyaluronate de sodium 0,050 - 0,200 g/100 g, NaCl 0,150 - 0,400 g/100 g, Na₂HPO₄*12H₂O 0,200 - 0,600 g/100 g, citrate de potassium*H₂O 0,010 - 0,060 g/100 g, KCl 0,050 - 0,200 g/100 g, CaCl₂*2H₂O 0,005 - 0,020 g/100 g, MgCl₂*6H2O 0,005 - 0,020 g/100 g, glycérol 0,050 - 0,900 g/100 g.

5. La composition selon l'une des revendications 1 à 4 comprenant : PVA 0,25 g, hyaluronate de sodium 0,180 g, NaCl 0,279 g, Na₂HPO₄*12H₂O 0,322 g, citrate de potassium*H₂O 0,031 g, KCl 0,103 g, CaCl₂*2H₂O 0,0089 g, MgCl₂*6H₂O 0,0092 g, glycérol 0,200 g, H₂O qs.

6. La composition selon l'une des revendications 1 à 5 consistant en : PVA 0,25 g, hyaluronate de sodium 0,180 g, NaCl 0,279 g, Na₂HPO₄12H2O 0,322 g, citrate de potassium*H₂O 0,031 g, KCl 0,103 g, CaCl2*2H2O 0,0089 g, MgCl2*6H2O 0,0092 g, glycérol 0,200 g, H₂O qs.

7. La composition selon l'une des revendications 1 à 6 pour utilisation comme substitut lacrymal.
